(19) Europäisches Patentamt
European Patent Office
Office européen des brevets

(11) Veröffentlichungsnummer: **0 034 783**
**B1**

(12) # EUROPÄISCHE PATENTSCHRIFT

(45) Veröffentlichungstag der Patentschrift: **02.03.83**

(51) Int. Cl.³: **C 07 D 249/08**, C 07 D 233/56

(21) Anmeldenummer: **81101055.2**

(22) Anmeldetag: **14.02.81**

(54) **Verfahren zur Herstellung von 1-Azolyl-3,3-dimethyl-1-phenoxybutan-2-olen.**

(30) Priorität: **26.02.80 DE 3007079**

(43) Veröffentlichungstag der Anmeldung: **02.09.81 Patentblatt 81/35**

(45) Bekanntmachung des Hinweises auf die Patenterteilung: **02.03.83 Patentblatt 83/9**

(84) Benannte Vertragsstaaten: **BE CH DE FR GB IT LI NL**

(56) Entgegenhaltungen:
**DE-A-2 333 354**

**CHEMICAL AND PHARMACEUTICAL BULLETIN Seiten 747–751 Japan M. SEKIYA et al.: »Formic Acid Reduction. IV. Barbituric Acid Derivatives. Reduction of 5-Aryl-methylenebarbituric Acids and Some Analogous Barbituric Acid Derivatives«.**

(73) Patentinhaber: **BAYER AG, Zentralbereich Patente, Marken und Lizenzen, D-5090 Leverkusen 1, Bayerwerk (DE)**

(72) Erfinder: **Kranz, Eckart, Dr., Am Acker 9, D-5600 Wuppertal-1 (DE)**

EP 0 034 783 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von 1-Azolyl-3,3-dimethyl-1-phenoxy-butan-2-olen

Die vorliegende Erfindung betrifft ein neues Verfahren zur Herstellung von bekannten fungizid wirksamen 1-Azolyl-3,3-dimethyl-1-phenoxy-butan-2-olen.

Es ist bereits bekannt geworden, daß man 1-Azolyl-3,3-dimethyl-1-phenoxy-butan-2-ole erhält, wenn man die entsprechenden Keto-Derivate in üblicher Weise reduziert, wie

(a) Mit Wasserstoff in Gegenwart eines Katalysators, z. B. Raney-Nickel, und in Gegenwart eines polaren Lösungsmittels, z. B. Methanol, bei Temperaturen von 20 bis 50°C; oder
(b) mit Aluminiumisopropylat in Gegenwart eines inerten organischen Lösungsmittels bei Temperaturen von 20 bis 120°C und anschließender Hydrolyse; oder
(c) mit komplexen Hydriden, z. B. Natriumborhydrid, in Gegenwart eines polaren Lösungsmittels, z. B. Methanol, bei Temperaturen von 0 bis 30°C und anschließender Hydrolyse, z. B. mit wäßriger Salzsäure; oder
(d) mit Formamidinsulfinsäure und Alkalihydroxid, z. B. Natriumhydroxid, in Gegenwart eines polaren Lösungsmittels, z. B. Ethanol, bei Temperaturen zwischen 20 bis 100°C (vgl. hierzu z. B. DE-OS 2 324 010 und DE-OS 2 333 354). Die genannten Verfahren sind in ihrer Durchführung zum Teil aufwendig und kostspielig.

Weiterhin ist bekannt geworden, daß man Ameisensäure-Triethylamin als 5 : 2-Additionsverbindung für Reduktionsreaktionen an C—C- und C—N-Doppelbindungen einsetzen kann, wie z. B. zur Reduktion *α,β*-ungesättigter Ketone, wobei die Carbonylgruppen selbst nicht angegriffen werden (vergleiche u. a. Chem. Pharm. Bull. 17, 747 (1969) und 18, 1530 (1970)).

Es wurde gefunden, daß man die bekannten 1-Azolyl-3,3-dimethyl-1-phenoxy-butan-2-ole der Formel

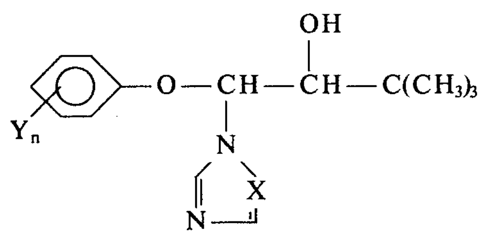

(I)

in welcher

X für ein Stickstoffatom oder die CH-Gruppe steht,
Y für Halogen, Phenyl, Phenoxy, Nitro, Alkyl, Alkoxy oder Cycloalkyl steht und
n für ganze Zahlen von 0 bis 3 steht,

erhält, wenn man 1-Azolyl-3,3-dimethyl-1-phenoxy-butan-2-one der Formel

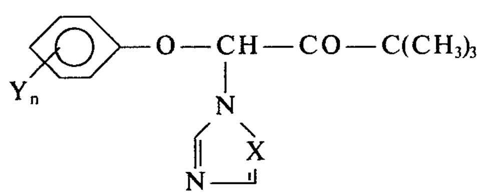

(II)

in welcher

X, Y und n die oben angegebene Bedeutung haben,

mit Ameisensäure-Triethylamin als 5 : 2-Additionsverbindung der Formel

$$5\ HCOOH \cdot 2\ N(C_2H_5)_3 \qquad (III)$$

erhitzt.

Es ist als überraschend zu bezeichnen, daß nach dem erfindungsgemäßen Verfahren die Endprodukte in verhältnismäßig guter Ausbeute erhalten werden, da aufgrund des Standes der Technik nicht zu erwarten war, daß sich die Carbonylgruppe mit dem erfindungsgemäßen Reagenz reduzieren läßt.

Das erfindungsgemäße Verfahren weist den Vorteil auf, daß sich mit dem Ameisensäure-Triethylamin

5 : 2-Additionsprodukt ein neues, leicht zu handhabendes und preiswertes Reduktionsmittel für Carbonylgruppen anbietet.

Die nach dem erfindungsgemäßen Verfahren herstellbaren 1-Azolyl-3,3-dimethyl-1-phenoxy-butan-2-ole sind durch die Formel (I) allgemein definiert. In dieser Formel steht Y vorzugsweise für Fluor, Chlor, Brom, Jod, Phenyl, Phenoxy, Nitro, geradkettiges oder verzweigtes Alkyl und Alkoxy mit jeweils 1 bis 4 Kohlenstoffatomen, sowie für Cycloalkyl mit 5 oder 6 Kohlenstoffatomen. X und der Index n haben vorzugsweise die in der Erfindungsdefinition angegebene Bedeutung.

Verwendet man beispielsweise 1-(4-Chlorphenoxy)-3,3-dimethyl-1-(1,2,4-triazol-1-yl)-butan-2-on als Ausgangsstoff, so kann der Reaktionsablauf durch das folgende Formelschema wiedergegeben werden:

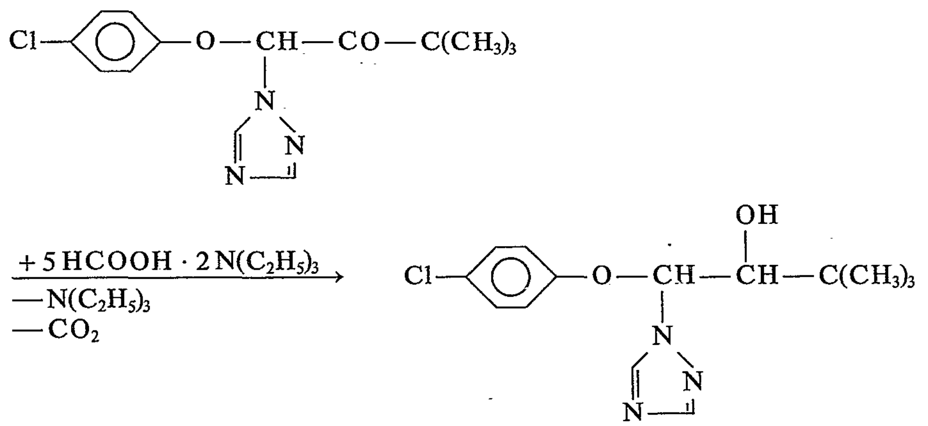


Die bei der Durchführung des erfindungsgemäßen Verfahrens als Ausgangsstoffe zu verwendenden 1-Azolyl-3,3-dimethyl-1-phenoxy-butan-2-one sind durch die Formel (II) allgemein definiert. In dieser Formel stehen X, Y und der Index n vorzugsweise für die Reste, die bereits im Zusammenhang mit der Beschreibung der erfindungsgemäß herstellbaren Stoffe der Formel (I) vorzugsweise für diese Substituenten genannt wurden.

Die 1-Azolyl-3,3-dimethyl-1-phenoxy-butan-2-one der Formel (II) sind bekannt (vergleiche DE-OS 2 105 490 und DE-OS 2 201 063).

Die erfindungsgemäße Umsetzung wird ohne Lösungsmittel durchgeführt.

Die Reaktionstemperaturen können bei der Durchführung des erfindungsgemäßen Verfahrens in einem größeren Bereich variiert werden. Im allgemeinen arbeitet man zwischen 80 und 200°C, vorzugsweise zwischen 100 und 160°C.

Bei der Durchführung des erfindungsgemäßen Verfahrens setzt man auf 1 Mol Keton der Formel (II) 1 bis 4 Mol Reduktionsmittel der Formel (III) ein. Die Isolierung der Endprodukte erfolgt in allgemein üblicher Art und Weise.

Die erfindungsgemäß herstellbaren Stoffe zeichnen sich bekanntermaßen durch sehr gute fungizide Wirksamkeit aus (vergleiche deutsche Offenlegungsschriften 2 324 010 und 2 333 354).

Das erfindungsgemäße Verfahren sei anhand der folgenden Herstellungsbeispiele erläutert.

## Herstellungsbeispiele

### Beispiel 1

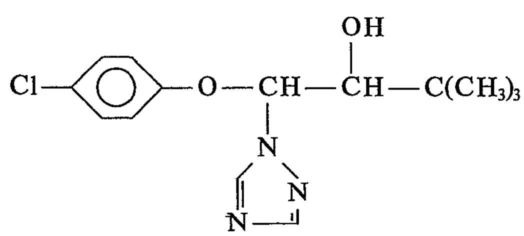

28,45 g (0,1 Mol) 1-(4-Chlorphenoxy)-3,3-dimethyl-1-(1,2,4-triazol-1-yl)-butan-2-on und 43,25 g (0,1 Mol) Ameisensäure-Triethylamin (5 : 2-Additionsverbindung) wurden 4,5 bis 5 Stunden auf 145 bis 150°C erhitzt.

Man läßt abkühlen und engt das Reaktionsgemisch im Vakuum ein. Der Rückstand wird in verdünnter Natronlauge aufgenommen und mit Methylenchlorid extrahiert. Die organische Phase wird mit Wasser gewaschen, über Natriumsulfat getrocknet und im Vakuum eingeengt. Der Rückstand wird in heißem

Cyclohexan aufgeschlämmt, heiß filtriert und getrocknet. Man erhält 18,2 g (63,6% der Theorie) 1-(4-Chlorphenoxy)-3,3-dimethyl-1-(1,2,4-triazol-1-yl)-butan-2-ol vom Schmelzpunkt 114—116°C.

Beispiel 2

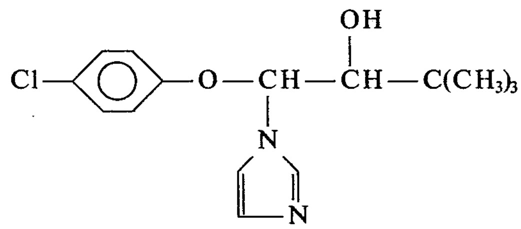

14 g (0,048 Mol) 1-(4-Chlorphenoxy)-3,3-dimethyl-1-(imidazol-1-yl)-butan-2-on und 61,8 g (0,143 Mol) Ameisensäure-Triethylamin (5 : 2-Additionsverbindung) werden 4 Stunden bei 145°C erhitzt. Man läßt abkühlen und engt das Reaktionsgemisch im Vakuum ein. Der Rückstand wird in verdünnter Natronlauge aufgenommen und mit Methylenchlorid extrahiert. Die organische Phase wird mit Wasser gewaschen, über Natriumsulfat getrocknet und im Vakuum eingeengt. Der Rückstand wird in heißem Cyclohexan aufgeschlämmt, heiß filtriert und getrocknet. Man erhält 7,9 g (56% der Theorie) 1-(4-Chlorphenoxy)-3,3-dimethyl-1-(imidazol-1-yl)-butan-2-on vom Schmelzpunkt 145–147°C.

In analoger Weise können die nachfolgenden Verbindungen der allgemeinen Formel

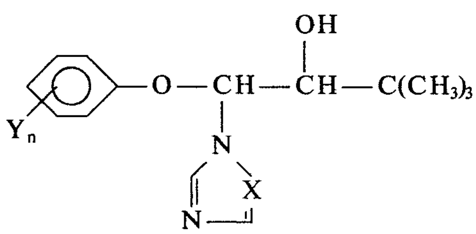

(I)

hergestellt werden:

| Bsp. Nr. | $Y_n$ | X | Fp (°C) |
|---|---|---|---|
| 3 | 4-$NO_2$ | N | 194–96 |
| 4 | 2,4-$Cl_2$ | N | 114–16 |
| 5 | 4-$C(CH_3)_3$ | N | 113–17 |
| 6 | 4-Br | N | 115–18 |
| 7 | 2,4,5-$Cl_3$ | N | 137–44 |
| 8 | 4-$CH_3$ | N | 123–27 |
| 9 | 2-Cl | N | 107–12 |
| 10 | 3,4-$(CH_3)_2$ | N | 133–35 |
| 11 | 2-$CH_3$, 4-Cl | N | 107–10 |
| 12 | 3-Cl | N | 114–15 |
| 13 | 4-F | N | 99–110 |
| 14 | — | N | 88–94 |
| 15 | 4- 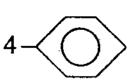 | N | 98–109 |
| 16 | 4- 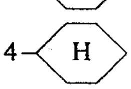 | N | 115–20 |

Fortsetzung

| Bsp. Nr. | $Y_n$ | X | Fp (°C) |
|---|---|---|---|
| 17 | 2-Cl, 4 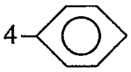 | N | 95–98 |
| 18 | 2,6-$Cl_2$, 4 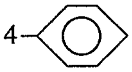 | N | 142–44 |
| 19 | 2,6-$Cl_2$ | CH | 95–102 |
| 20 | 2,4-$Cl_2$ | CH | 101–09 |
| 21 | 4-F | CH | 103–05 |
| 22 | 3-Cl | CH | 102–06 |
| 23 | — | CH | 99–105 |
| 24 | 4 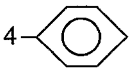 | CH | 136–40 |
| 25 | 4-$C(CH_3)_3$ | CH | 145–50 |
| 26 | 4-Br | CH | 173–74 |
| 27 | 4 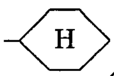 | CH | 134–40 |
| 28 | 2-Cl, 4 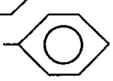 | CH | 86–95 |
| 29 | 2,6-$Cl_2$, 4 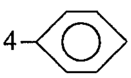 | CH | 110–13 |
| 30 | 4-J | CH | 192–94 |
| 31 | 2-F | CH | 118–30 |
| 32 | 3-Br | CH | 125–27 |
| 33 | 3-$CH_3$ | CH | 92–94 |

**Patentansprüche**

1. Verfahren zur Herstellung von 1-Azolyl-3,3-dimethyl-1-phenoxy-butan-2-olen der Formel

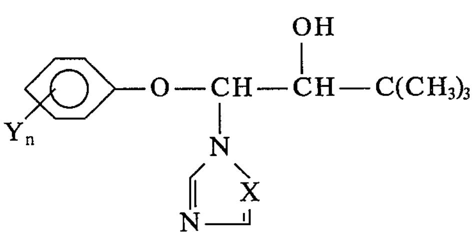

(I)

in welcher

X für ein Stickstoffatom oder die CH-Gruppe steht,
Y für Halogen, Phenyl, Phenoxy, Nitro, Alkyl, Alkoxy oder Cycloalkyl steht und
n für ganze Zahlen von 0 bis 3 steht,

dadurch gekennzeichnet, daß man 1-Azolyl-3,3-dimethyl-1-phenoxy-butan-2-one der Formel

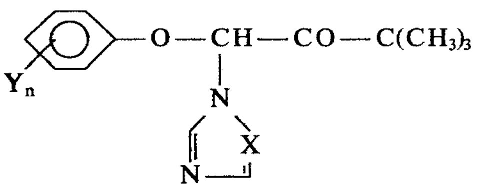

(II)

in welcher

X, Y und n die oben angegebene Bedeutung haben,

mit Ameisensäure-Triethylamin als 5 : 2-Additionsverbindung der Formel

$$5\ HCOOCH \cdot 2\ N(C_2H_5)_3 \qquad (III)$$

erhitzt.

2. Verfahren gemäß Anspruch 1, dadurch gekennzeichnet, daß man die Reaktion im Temperaturbereich zwischen +80 und 200°C durchführt.

3. Verfahren gemäß Ansprüchen 1 und 2, dadurch gekennzeichnet, daß man die Reaktion im Temperaturbereich zwischen 100 und 160°C durchführt.

4. Verfahren gemäß Anspruch 1, dadurch gekennzeichnet, daß man auf 1 Mol 1-Azolyl-3,3-dimethyl-1-phenoxy-butan-2-on der Formel II 1 bis 4 Mole der Additionsverbindung der Formel III einsetzt.

**Claims**

1. Process for the preparation of 1-azolyl-3,3-dimethyl-1-phenoxy-butan-2-ols of the formula

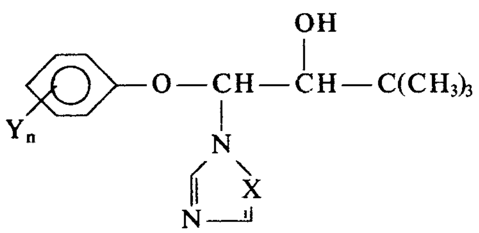

(I)

in which

X represents a nitrogen atom or the CH group,
Y represents halogen, phenyl, phenoxy, nitro, alkyl, alkoxy or cycloalkyl and
n represents integers from 0 to 3,

characterised in that 1-azolyl-3,3-dimethyl-1-phenoxy-butan-2-ones of the formula

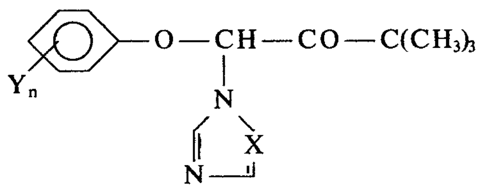

(II)

in which

X, Y and n have the meaning indicated above,

are heated with formic acid/triethylamine in the form of a 5 : 2 addition compound of the formula

$$5\ HCOOH \cdot 2\ N(C_2H_5)_3 \qquad (III)$$

2. Process according to Claim 1, characterised in that the reaction is carried out in the temperature range betwen +80 and 200°C.

3. Process according to Claims 1 and 2, characterised in that the reaction is carried out in the temperature range between 100 and 160°C.

4. Process according to Claim 1, characterised in that 1 to 4 moles of the addition compound of the formula III are used per mole of 1-azolyl-3,3-dimethyl-1-phenoxy-butan-2-one of the formula II.

**Revendications**

1. Procédé de préparation de 1-azolyl-3,3-diméthyl-1-phénoxy-butane-2-ols de formule:

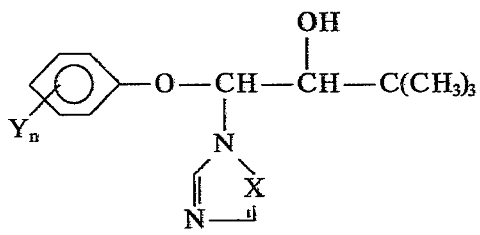

(I)

dans laquelle

X représente un atome d'azote ou le groupe CH,
Y représente un halogène, un groupe phényle, phénoxy, nitro, alkyle, alcoxy ou cycloalkyle, et
n représente un nombre entier de 0 à 3,

caractérisé en ce qu'on chauffe des 1-azolyl-3,3-diméthyl-1-phénoxy-butane-2-ones de formule:

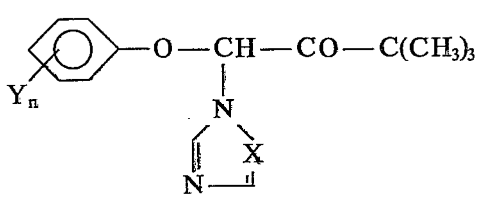

(II)

dans laquelle

X, Y et n ont les significations indiquées ci-dessus,

avec l'acide formique et la triéthylamine sous la forme du composé d'addition 5 : 2 de formule:

$$5\ HCOOH \cdot 2\ N(C_2H_5)_3 \qquad (III)$$

2. Procédé selon la revendication 1, caractérisé en ce que l'on effectue la réaction dans l'intervalle de température de +80 à 200°C.
3. Procédé selon les revendications 1 et 2, caractérisé en ce que l'on effectue la réaction dans l'intervalle de température de 100 à 160°C.
4. Procédé selon la revendication 1, caractérisé en ce que, pour 1 mole de 1-azolyl-3,3-diméthyl-1-phénoxy-butane-2-one de formule (II), on utilise 1 à 4 moles du composé d'addition de formule (III).